# EUROPEAN PATENT SPECIFICATION

(11) **EP 3 648 716 B1**
(45) Date of publication and mention of the grant of the patent: **02.08.2023**
(21) Application number: 17742956.0
(22) Date of filing: 07.07.2017
(51) Int. Cl.: A61F 13/472, A61F 13/476, A61F 13/56, A61F 13/15

(54) **ABSORBENT ARTICLE AND METHOD FOR MANUFACTURING AN ABSORBENT ARTICLE**
SAUGFÄHIGER ARTIKEL UND VERFAHREN ZUR HERSTELLUNG DES SAUGFÄHIGEN ARTIKELS
ARTICLE ABSORBANT ET PROCÉDÉ DE FABRICATION D'UN ARTICLE ABSORBANT

(43) Date of publication of application: 13.05.2020
(73) Proprietor: Essity Hygiene and Health Aktiebolag, 405 03 Göteborg (SE)
(72) Inventor: RÖNNBERG, Peter, 405 03 Göteborg (SE); BLOMSTRÖM, Philip, 405 03 Göteborg (SE)
(74) Representative: Nederlandsch Octrooibureau
(86) International application number: PCT/EP2017/067071
(87) International publication number: WO 2019/007529

(56) References cited:
- EP-A1- 1 138 294
- US-A- 5 401 268
- US-A1- 2004 138 636
- US-A1- 2005 283 131

## Description

### TECHNICAL FIELD

The invention relates to an absorbent article comprising a main body having a liquid-permeable topsheet, a liquid-impermeable backsheet and an absorbent core sandwiched between said topsheet and said backsheet, said main body being arranged along a longitudinal axis and a transversal axis extending in a perpendicular direction in relation to the longitudinal axis, said main body defining a front portion, a back portion and a crotch portion; wherein said article comprises a first wing and a second wing extending outwardly from said crotch portion of the main body in opposite directions in an asymmetric manner with reference to the longitudinal axis, and generally along said transversal axis, said wings being provided with fastening means configured for fastening said wings to a garment when being folded over said main body and towards said longitudinal axis.

The invention also relates to a method for manufacturing an absorbent article having a longitudinal extension along a longitudinal axis and a transverse extension along a transverse axis, said article defining a front portion, a back portion and a crotch region. Furthermore, the method comprises the steps of: forming a main body by providing a liquid-permeable topsheet, providing a liquid-impermeable backsheet and sandwiching an absorbent body between said topsheet and said backsheet; forming a first wing and a second wing configured so as to extend outwardly from said main body in opposite directions and generally along said transversal axis; and providing said wings with fastening means for fastening said wings to a garment when being folded over said main body and towards said longitudinal axis.

### BACKGROUND OF THE INVENTION

Absorbent articles, for example in the form of sanitary napkins and panty liners, are well known. The general purpose of such absorbent articles is to absorb, distribute and store various types of body exudates while providing a high level of comfort and sense of dryness to the wearer during use of the absorbent article. Also, such absorbent articles are arranged to prevent the wearer from getting the clothes soiled by body exudates.

A conventional sanitary napkin is normally designed with a main body which comprises a liquid-permeable topsheet, a liquid-impermeable backsheet and an absorbent core which is sandwiched between the topsheet and the backsheet. The main body is arranged along a longitudinal axis and a transversal axis which extends in a perpendicular direction in relation to the longitudinal axis.

Furthermore, absorbent articles in the form of sanitary napkins are often provided with two wings, i.e. two wing-shaped fastening elements, extending outwardly from the main body in opposite directions and generally along the transversal axis. The purpose of the wings is to allow fastening of the sanitary napkin to an undergarment of a user. To this end, the wings are arranged to be folded over the main body and towards said longitudinal axis. In order to allow fastening of the sanitary napkin to the undergarment, each wing is provided with fastening means such as an adhesive material. In this manner, the sanitary napkin can be attached and held in place in the undergarment during use.

According to prior art, the wings of a sanitary napkin can be formed in a symmetrical manner, i.e. so that the entire sanitary napkin can be said to define a contour which is symmetrical with reference to its longitudinal axis. Alternatively, it is also known that the wings can be formed in an asymmetrical manner with reference to the longitudinal axis.

A previously known absorbent article having asymmetrical wings of the above-mentioned type is known from the patent document US 5401268. This document shows a sanitary napkin having two wings which are arranged asymmetrically along opposite sides of a longitudinal edge of the napkin.

The wings disclosed in US 5401268 are formed of two triangular pieces which comprise semi-circular indentations on the base of said pieces, and also a rounded cutout at the top of each triangular piece. The purpose of the wings shown in US 5401268 is to provide a relatively large surface for fixing the sanitary napkin to an undergarment.

Even though the article disclosed in US 5401268 fulfills the basic requirements regarding absorbent articles provided with wing-shaped fastening elements, there is a need for further improvements within this field of technology. Firstly, there is a requirement that the wings must be configured to attach the napkin to an undergarment in a secure manner, in particular so as to improve the comfort for the wearer. This means that the attachment of the wing-shaped elements must not cause any unwanted shearing forces in the undergarment, or any other causes of instability of the fastening of the article to an undergarment. Also, there are continuing efforts made in order to provide more cost-effective manufacturing methods for the products of this type, including methods for forming the wings.

Consequently, there is a need for further improvements within the relevant field of technology.

### SUMMARY OF THE INVENTION

In accordance with the invention, there is provided an absorbent article with the purpose of solving the above-mentioned problems related to prior art within this field. In particular, the article is configured so as to provide a secure and stable attachment of a sanitary napkin to an undergarment, in order to provide a high level of comfort for the wearer while also providing the required absorbent properties. Also, the invention aims at contributing to a cost-effective manufacturing process for sanitary napkins.

In accordance with the invention, this object is obtained by means of an absorbent article as defined in claim 1.

The invention provides certain advantages. In particular, the invention fulfills the requirements on sanitary napkins to be securely fitted and held in place in an undergarment of a wearer.

According to an embodiment, the magnitude of said first angle is chosen so that it is between 20° - 55°. According to a further embodiment, the magnitude of said second angle is chosen so that it is between 70° - 110°.

According to a further embodiment, said wings extend generally along a further common straight line which defines a third angle with respect to said transversal axis which exceeds 0° but which is less than 45°.

According to an embodiment, the outer edge of each wing is generally straight and parallel to the longitudinal axis.

According to an embodiment, said straight line extends between a first edge mid-point on the outer edge of the first wing and a second edge mid-point on the outer edge on the second wing, said mid-points being positioned halfway along said outer edges and defining outermost points of said wings.

According to a further embodiment, a first distance, as defined between the outer edge of each wing and the longitudinal axis, is in the range of 40-100 mm. This means that the invention can be used for virtually all types of sanitary napkins and similar absorbent articles.

According to an embodiment, at least a part of each longitudinal side of the main body defines a first wing folding edge where each wing meets the main body and along which each wing is folded during manufacturing of the article.

According to an embodiment, a second distance, as defined between the first wing folding edge and the corresponding outer edge is in the range of 20-60 mm.

According to an embodiment, a second wing folding edge is defined where each wing can be folded during use of the article, said second wing folding edge having a length which is within the interval 10-80% of the full length of the main body, and preferably 25-65% of the full length of the main body.

According to a further embodiment, each longitudinal side of the absorbent core at least partly defines a core edge which is generally parallel to the longitudinal axis.

Also, according to a further embodiment, a third distance, as defined between the core edge and the corresponding outer edge is in the range of 29-69 mm. According to a further embodiment, a fourth distance is defined between the core edge and the longitudinal axis is in the range of 10-50 mm.

According to a further embodiment, the absorbent core generally follows the outer edge of the main body and defines an edge region of the main body which is generally equally wide along the entire periphery of the absorbent core.

According to an embodiment, said fastening means is the form of one or more areas which supports an adhesive material on each of said wing.

According to a further embodiment, the areas are generally rectangular and positioned on each wing so that they are displaced in relation to each other in the longitudinal direction of the article.

According to another embodiment, the areas are arranged and so that they extend along generally the entire transversal length of each corresponding wing. This contributes to a firm and stable attachment of the wings of the sanitary napkin to an undergarment by the positioning of the available adhesive.

According to the invention, said wings are formed with an area between said fastening means and a further edge which constitutes a grip tab. In this manner, the wings can be handled easily during fastening on an undergarment and during removal from the undergarment.

According to a further embodiment, the article comprises a second fastening means positioned along the main body on a garment-facing side of said backsheet. This contributes to the attachment of the article in the undergarment.

Furthermore, the above-mentioned object of the invention is obtained by means of a method for manufacturing an absorbent article as defined in claim 14.

Further advantages and advantageous features of the invention are disclosed in the following description and in the dependent claims.

### BRIEF DESCRIPTION OF THE DRAWINGS

The invention will be described in greater detail below with reference to the figures shown in the appended drawings.
- Figure 1: shows a view from above of an absorbent article in the form of a sanitary napkin according to an embodiment of the invention,
- Figure 2: shows a cross-sectional view of the sanitary napkin,
- Figure 3: shows a view from above of the sanitary napkin shown in Figures 1 and 2 but in a condition as manufactured,
- Figure 4: shows a further embodiment of the invention,
- Figure 5: is a schematic view of an embodiment in which certain geometric properties of the invention are disclosed,
- Figure 6: is a simplified view of a further embodiment which merely represent apossible exemplary execution and is not part of the present invention,
- Figure 7: is a simplified view of yet another embodiment which merely represent a possible exemplary execution and is not part of the present invention,
- Figure 8: is a simplified view of yet another embodiment which merely represent a possible exemplary execution and is not part of the present invention, and
- Figure 9: is a simplified view of yet another embodiment which merely represent a possible exemplary execution and is not part of the present invention.

### DESCRIPTION OF EMBODIMENTS

Different aspects of the present disclosure will be described more fully hereinafter with reference to the enclosed drawings. The embodiments disclosed herein can, however, be realized in many different forms and should not be construed as being limited to the aspects set forth herein.

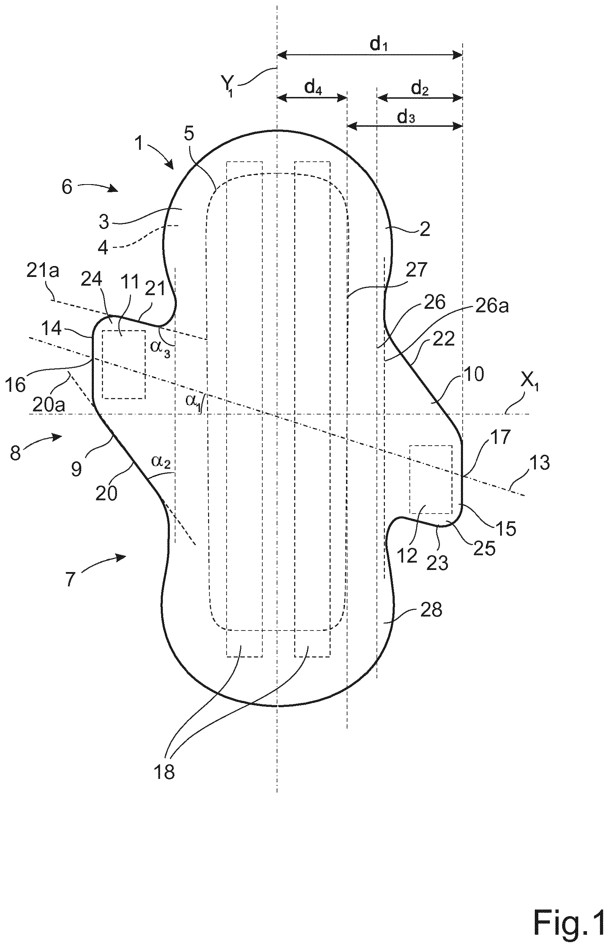

With initial reference to Fig. 1, there is shown a view from above of an absorbent article in the form of a sanitary napkin 1 manufactured in accordance with an embodiment of the invention. The sanitary napkin 1 is based on an absorbent structure for absorbing body exudates from a wearer in order to provide a dry, comfortable and odor-free feeling for the wearer.

From Figure 1 it can be understood that the sanitary napkin 1 comprises a main body 2 with a liquid-permeable topsheet 3 and a liquid-impermeable backsheet 4. The sanitary napkin 1 also comprises an absorbent core 5 which is sandwiched between the topsheet 3 and the backsheet 4. The topsheet 3 is arranged at the surface of the sanitary napkin 1, i.e. the side facing the wearer. The backsheet 4 is arranged at the underside of the sanitary napkin 1, i.e. the side facing an undergarment (not shown) of the wearer. Furthermore, both the topsheet 3 and the backsheet 4 extend laterally outside of the absorbent core 5 along the whole perimeter of the main body 2.

The absorbent core shown in Figure 1 has a substantially rectangular design. However, the invention is not limited to this particular design but can be formed in generally any geometric form within the scope of the invention.

According to further embodiments, one or more additional layers may be provided in the absorbent article 1. For example, an acquisition layer may be arranged between the absorbent core 4 and the topsheet 3. Such an additional layer can for example be in the form of an airlaid layer, a spunlace layer, a high-loft, foam or any other type of material layer which may be used in an absorbent article in order to act as a liquid acquisition and absorption layer. The acquisition layer is adapted to quickly receive and temporarily store discharged liquid before it is absorbed by the absorbent core. Such acquisition layer may be composed of for example airlaid nonwoven, spunlace nonwoven, high loft nonwoven or foam materials. An airlaid nonwoven can be produced with fluff, wood pulp, and here the fluff fibres are dispersed into a fast moving air stream and condensed onto a moving screen by means of pressure and vacuum. The web can be bonded with resin and/or thermal plastic resin dispersed with the pulp. The web can be thermobonded (by heat), latex bonded (by adhesive) or multibonded (a combination of thermos and latex bonding) or mechanically bonded (high compression and temperature, bonding by hydrogen). The grammage of the airlaid nonwoven may be for example from 50 to 100 gsm.

The topsheet 3, backsheet 4 and the absorbent core 5 may consist of any materials suitable for their particular purposes, as will be discussed in further detail below. Also, the above-mentioned layers 3, 4, 5 may be connected to each other by any conventional means such as by means of an adhesive, heat bonding or ultrasonic bonding.

Furthermore, and although not shown in the drawings, the sanitary napkin 1 can be configured in a manner having different material layers, densities or material components as seen along a horizontal plane of the sanitary napkin 1, i.e. in addition to being configured with different layers in the vertical direction.

As shown in Figure 1, the sanitary napkin 1 has a longitudinal extension along a longitudinal axis Y1 and a transverse extension along a transverse axis X1. Furthermore, the sanitary napkin 1 can be said to be divided into a front portion 6, a back portion 7 and a crotch portion 8. The front portion 6 is intended to be oriented in a direction towards the wearer's belly during use of the sanitary napkin 1.

Furthermore, according to the embodiment, the sanitary napkin 1 is formed with two fastening wings 9, 10, or fastening tabs, which are configured for fastening the napkin 1 to an undergarment of the wearer. More precisely, the sanitary napkin 1 comprises a first wing 9 and a second wing 10, each of which extends outwardly from the main body 2 and in opposite directions. The wings 9, 10 extend generally along the direction of the transversal axis X1. Also, the wings 9, 10 are provided with fastening means 11, 12, which according to the embodiment are in the form of one or more adhesive areas which are configured for fastening the wings 9, 10 to the undergarment when the wings 9, 10 are folded under the main body 2 in a direction towards the longitudinal axis Y1.

As indicated in Figure 1, the two wings 9, 10 are arranged in an asymmetrical manner so that they extend generally along a common straight line 13 which defines an angle α1 with respect to the transversal axis X1. According to an embodiment, the magnitude of the angle α1 exceeds 0° but is less than 45°, preferably less than 35°, most preferably less than 25°. It should be noted that the wings 9, 10 can be arranged so that they are displaced in relation to each other in the longitudinal direction of the sanitary napkin 1.

Also, each wing 9, 10 has an outer edge 14, 15 which is generally straight and parallel to the longitudinal axis Y1. This means that each one of the outer edges 14, 15 is cut off or otherwise shaped so as to form an outer, straight side edge which terminates each wing 9, 10 and which also is generally parallel to the longitudinal axis Y1.

In order to clearly define the extension of the above-mentioned straight line 13 shown in Figure 1, there is defined a first edge mid-point 16 on the outer edge 14 of the first wing 9.

Also, a second edge mid-point 17 is defined on the outer edge 15 of the second wing 10. These mid-points 16, 17 are defined in a manner so that they are positioned halfway along the length of each corresponding outer edge 14, 15 and also define outermost points of each one of the wings 9, 10. This means that the above-mentioned straight line 13 can be defined as extending through the two mid-points 16, 17.

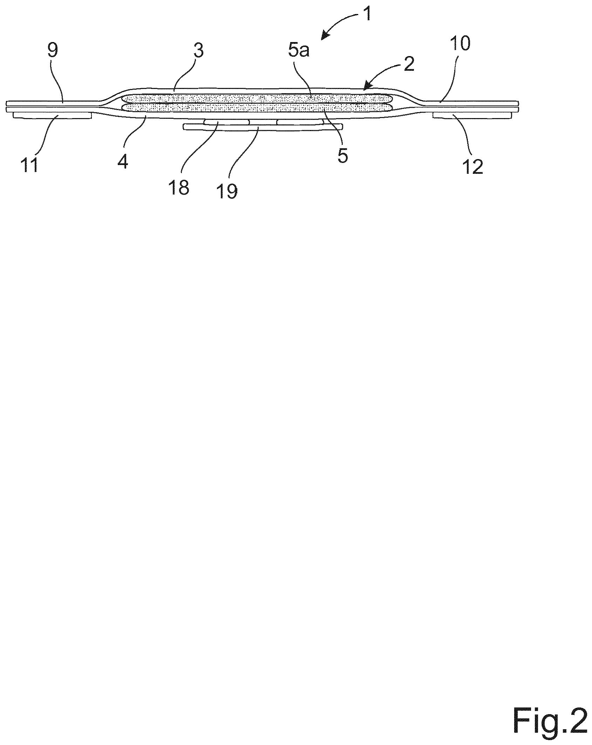

Figure 2 is a cross-sectional view as seen along the straight line 13 shown in Figure 1. The absorbent core 5 is sandwiched between the topsheet 3 and the backsheet 4. As mentioned above, an acquisition layer 5a, suitably of airlaid material, can be arranged between the absorbent core 5 and the topsheet 3. Also, the two wings 9, 10 are formed so as to extend in opposite directions from the main body 2. According to the embodiment, each of the wings 9, 10 is provided with fastening means in the form of at least one adhesive area 11, 12, which is applied on the underside of each wing 9, 10, i.e. on the side which does not face the wearer. Furthermore, the main body 2 is also provided with further fastening means 18 for the purpose of fastening the sanitary napkin 1 onto an inner surface of an undergarment. Such further fastening means 18 may be in the form of one or several adhesive sections 18 which are arranged on the rear side of the backsheet 4 and which are arranged to extend along the longitudinal direction of the sanitary napkin 1.

As shown in Figure 2, the adhesive sections 18 are covered with a release paper layer 19 when the sanitary napkin 1 is in its non-used condition. When the sanitary napkin 1 is to be used, the release paper layer 19 is removed by the user so that the sanitary napkin 1 can be fastened to the undergarment.

According to alternative embodiments, the sanitary napkin 1 can be provided with various types of fastening means in the form of frictional fasteners, mechanical fasteners such as a hook-and-loop fastener, or combinations of different types of fasteners, as previously known.

The properties and design of the various layers 3, 4, 5 which form part of the sanitary napkin 1 will now be described more in detail, with reference primarily to Figures 1 and 2.

According to an embodiment, the topsheet 3 is formed by a fluid permeable nonwoven fabric or film which is made of thermoplastic synthetic fibers. The topsheet 3 is sufficiently liquid-permeable to allow discharged body fluids to penetrate through the thickness of the topsheet 3. Also, the topsheet 3 is suitably manufactured from a material which is compliant and soft-feeling to the skin of the wearer.

According to different embodiments, the topsheet 3 may be manufactured from various web materials such as for example nonwoven films, foams, or combinations of the above-mentioned materials. The topsheet 3 may also be perforated.

Furthermore, the backsheet 4 is constituted by a liquid-impermeable and breathable layer such as a polymeric film, for example a film of polyethylene or polypropylene. According to different embodiments, the materials which can be used for the backsheet 4 include thin and flexible fluid impermeable plastic films, or fluid impermeable nonwoven materials, fluid impermeable foams and fluid impermeable laminates.

According to the embodiment shown in the drawings, the backsheet 4 is formed by a single layer, but can alternatively be formed by a multi-layered structure, i.e. a laminate, wherein at least one layer is fluid impermeable. Furthermore, the backsheet 4 can optionally be elastic in any direction. Also, backsheet materials which are not fully liquid impermeable but only resistant to fluid penetration may be used, particularly in cases where relatively small amounts of body exudates are expected to be absorbed by the sanitary napkin 1.

According to further embodiments, the backsheet 4 may be breathable, implying that air and vapor may pass through the backsheet 4. Furthermore, the backsheet 4 may optionally have an outer, garment-facing surface of a textile material such as nonwoven.

Furthermore, the wings 9, 10 are formed in a manner in which the topsheet 3 and the backsheet 4 are laminated and formed with the wings 9, 10 being integrated as shown in the drawings. This means that the generally straight outer edges 14, 15 are cut in the desired form during a manufacturing process for the sanitary napkin 1. According to an alternative embodiment, which is not shown in the drawings, the wings can be manufactured separately and then supplied and finally attached to the main body during a final manufacturing process.

Furthermore, according to the embodiment shown in Figures 1 and 2, the sanitary napkin 1 comprises an absorbent core 5 which is formed by one or more layers comprising fibres of cellulosic fluff pulp. According to alternative embodiments, the absorbent core 5 can be made up of any suitable absorbent or fluid-absorbing material as known in the art, for example foam, fiber waddings and similar materials. As mentioned above, different types of material layers can be used, alone or in combination, for example airlaid, spunlace and high loft material, depending on the required properties and the field of use of the sanitary napkin 1 in question.

According to one embodiment, the absorbent article is a liner, in which case the absorbent core may only be made of one thin material, for example a nonwoven layer.

According to a further embodiment, the absorbent core 5 comprises a suitable amount of superabsorbent particles. Such superabsorbent material is well known in the field of absorbent articles, and is constituted by a water-swellable and water-insoluble material which is capable of absorbing large quantities of fluid upon formation of a hydrogel. Normal superabsorbent materials are capable of absorbing fluids of at least 10 times its own weight. According to an embodiment, the amount of said superabsorbent particles is at least 10% by weight, i.e. in relation to the total weight of the absorbent core 5.

The superabsorbents are mixed into the material of the absorbent core 5. The absorbent core 5 may further incorporate components for improving the properties of the absorbent core 5. Some examples of such components are binder fibers, fluid-dispersing materials, fluid acquisition materials, etc. as known in the art. According to an embodiment, a mixture of cellulose fluff pulp and superabsorbent articles is mixed in a generally homogeneous manner throughout the entire absorbent core 5.

According to further embodiments, the absorbent core 5 may be a homogeneous structure or may be a layered structure with laminates of the same or different materials. The absorbent layers may have uniform thickness or may vary in thickness in different parts of the layers. Also, the basis weight and composition of the absorbent core 1 may vary within such absorbent layers.

Furthermore, as known by the skilled person, the various layers of the sanitary napkin 1 can suitably be attached by means of layers of adhesive material. Such adhesive layers are not shown in the drawings.

With reference again to Figure 1, it can be noted that the first wing 9 is formed by a second edge section 20 which extends from a point along the perimeter of the main body 2 positioned in the back portion 7 of the sanitary napkin 1, and to the first outer edge 14. The first wing 9 also includes a third edge section 21 which extends from a point along the perimeter of the main body 2 in the front portion 6 of the sanitary napkin 1, and extends further to the first outer edge 14.

The second edge section 20 extends generally along an imaginary straight line 20a which defines a further angle α2 in relation to the longitudinal axis Y1, as indicated in Figure 1. Also, the third edge section 21 extends generally along a further imaginary straight line 21a which defines yet another angle α3 in relation to the longitudinal axis Y1. More precisely, and as shown in Figure 1, both the second edge section 20 and third edge section 21 extend in a manner in which at least an essential part of each section 20, 21 is generally straight and can be said to be aligned with each one of the two above-mentioned straight lines 20a, 21a.

As shown in Figure 1, the first wing 9 has a first edge section 20 extending generally along a first straight line 20a and a second edge section 21 extending generally along a second straight line 21a. The first line 20a defines an angle α2 with respect to the longitudinal axis Y1 and the second line 21a defines a further angle α3 with respect to the longitudinal axis Y1. Also, the magnitude of said angles α2, α3 is chosen so that α2<α3. According to an embodiment, the magnitude of the angle α2 is chosen so that it is within the interval 20°-55°. According to a further embodiment, the magnitude of the angle α3 is chosen so that it is within the interval 70°-110°.

The second wing 10 is arranged in a similar manner as the first wing 9 but is reversed (i.e. "upside-down") as compared with the first wing 9, i.e. with a fourth edge section 22 extending from the front portion 6 and a fifth edge section 23 extending from the back portion 7, i.e. so that the second outer edge 15 is situated between the fourth edge section 22 and the fifth edge section 23. The fourth edge section 22 and the fifth edge section 23 of the second wing 10 can be said to define the same type of arrangement as described above with reference to the straight lines 20a, 21a and the angles α2, α3 of the first wing 9.

According to the shown embodiment, the wings 9, 10 are arranged in a generally parallell manner, while being connected to the main body 2 in a slightly displaced manner. Consequently, the wings 9, 10 form an asymmetric design due to the design of the wings 9, 10 and also due to the fact that they extend generally along the above-mentioned common straight line 13 which defines an angle α1 with respect to the transversal axis X1.

As shown in Figure 1, the angle α3 is greater than 90°, which means that a relatively small area 24 is formed between the adhesive area 11 and the third edge section 21, which area 24 is not covered with any adhesive. This area 24 forms a grip tab which simplifies gripping and handling of the wings 9, 10 during fastening of the sanitary napkin 1 in an undergarment, and also during removal of the sanitary napkin 1 from the undergarment.

In the following, certain dimensions and measurements relating to an embodiment of the invention will now be described with reference to Figure 1. As indicated, a first distance d1, as defined between the outer edge 15 of each wing (here shown only with reference to the second wing 10) and the longitudinal axis Y1 is in the range of 40-100 mm, preferably 50-90 mm, most preferably 60-90 mm. According to a particular embodiment, the first distance d1 is approximately 77 mm. This corresponds to the distance from the centre of the absorption body 4 to the outer edge 14, 15 of each wing 9, 10. Furthermore, a first wing folding edge 26 is defined as a part of the longitudinal side of the main body 2 where each wing 9, 10 meets the main body 2 and along which each wing 9, 10 can be folded when the sanitary napkin 1 has been manufactured. The first wing folding edge 26 as shown in Figure 1 is defined as a line which is parallel to the longitudinal axis Y1.

Consequently, the first wing folding edge 26 is defined as part of the longitudinal side of the main body 2 where each wing 9, 10 meets the main body 2 and along which each wing 9, 10 is folded when the absorbent article 1 is manufactured.

As mentioned, the first wing folding edge 26 defines a line along which the corresponding wing 10 can be folded during manufacturing of the sanitary napkin 1. During use of the sanitary napkin 1, however, the wings 9, 10 are normally folded along a line which is adapted to the size of the the undergarment. Consequently, as also shown in Figure 1, a second wing folding edge 26a is defined as a line along which each wing 9, 10 can be expected to be folded during use of the sanitary napkin 1. According to embodiments, the second wing folding edge 26a is configured so that the length along which it is folded is within the interval 10-80% of the full length of the main body, and preferably 25-65% of the full length of the main body, depending on the size and the desired properties of the sanitary napkin 1.

A second distance d2 can be defined between the first wing folding edge 26 and the corresponding outer edge 18 of the corresponding wing 10, said second distance d2 being in the range of 20-60 mm, preferably 25-50 mm, most preferably 24-45 mm. According to a particular embodiment, the second distance d2 is approximately 39 mm. A further way of defining the magnitude of the second distance d2 is by defining it in relation to the full width of the sanitary napkin 1. According to embodiments, the ratio between the second distance d2 and the full width is in the range of 12-39%, preferably 16-32%, most preferably 16-29%.

Furthermore, a core edge 27 can be defined along at least a part of each longitudinal side of the absorbent core 5. As shown in Figure 1, the core edge 27 is generally parallel to the longitudinal axis Y1. Also, a third distance d3, as defined between the core edge 27 and the outer edge 18 of the corresponding wing 10, is in the range of 29-69 mm, preferably 34-65 mm, most preferably 34-61 mm. According to a particular embodiment, the third distance d3 is approximately 49 mm. A further way of defining the magnitude of the third distance d3 is by defining it in relation to the full width of the sanitary napkin 1. According to embodiments, the ratio between the third distance d3 and the full width is in the range of 18-45%, preferably 21-42%, most preferably 21-40%.

Also, a fourth distance d4 is defined between the core edge 27 and the longitudinal axis Y1, said fourth distance d4 being in the range of 10-50 mm, preferably 15-45 mm, most preferably 20-40 mm. According to a particular embodiment, the fourth distance d4 is approximately 29 mm. A further way of defining the magnitude of the fourth distance d4 is by defining it in relation to the full width of the sanitary napkin 1. According to embodiments, the ratio between the fourth distance d4 and the full width is in the range of 6-33%, preferably 9-29%, most preferably 12-25%.

Also, the core edge 27 is according to the embodiment shown in the drawings generally parallel with the longitudinal axis Y1. In other embodiments, however, the core edge can be non-parallel in relation to the longitudinal axis Y1. In such cases, the above-mentioned distances d3 and d4 will be dependent on the position along the longitudinal axis Y1.

Furthermore, the absorbent core 5 is formed with a periphery which generally follows the outer edge of the main body 2 and defines an edge region 28 along the entire periphery of the main body 2.

Also, the fastening means 11, 12 which is arranged on each one of the wings 9, 10 is according to an embodiment in the form of an area which supports an adhesive material. These adhesive areas 11, 12 are generally rectangular and positioned on each wing 9, 10 in a manner so that they are displaced in relation to each other in the longitudinal direction of the article 1, and also so that they extend along generally the entire transversal length of each corresponding wing 9, 10.

The arrangement of the fastening means 11, 12 according to the invention provides an advantage in that it increases the stability of each wing 9, 10. Also, the sanitary napkin 1 can be positioned and attached to an undergarment in a secure and comfortable manner, without any shearing forces acting on the undergarment, which otherwise could be detrimental to the comfort of the wearer.

Furthermore, due to the fact that the third edge 21 is arranged with an angle α3 with respect to the longitudinal direction of the main body 2 which is more than 90°, a grip tab 24, 25 - which does not support any adhesive covering - is formed in each wing 9, 10. In this manner, the handling of the wings 9, 10 is further simplified. Also, the comfort and stability of the sanitary napkin 1 during use is improved.

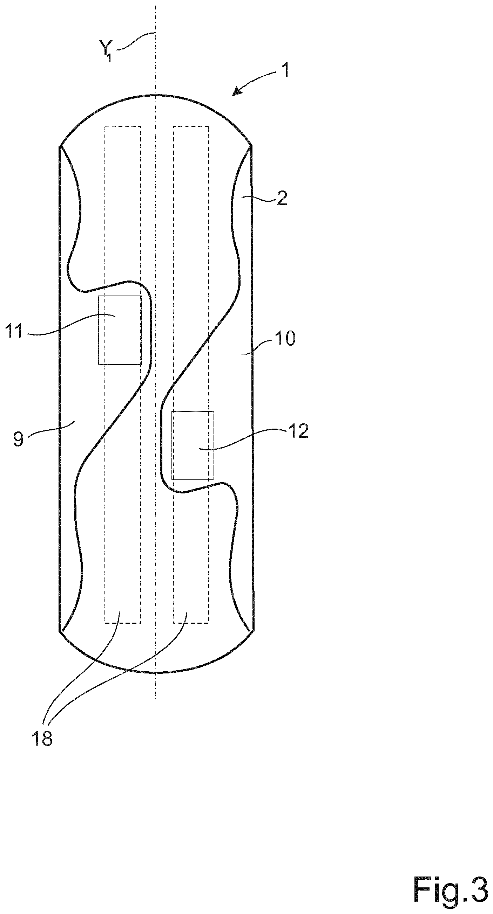

Figure 3 shows the sanitary napkin according to Figures 1 and 2 but in a condition as manufactured, i.e. with the wings 9, 10 being folded. Not all components and features of the sanitary napkin 1 which are shown in Figures 1 and 2 are indicated with reference numerals in Figure 3.

As indicated in Figure 3, the main parts of the adhesive areas 11, 12 are located close to the longitudinal axis Y1. Also, the adhesive areas 11, 12 are displaced in relation to each other along the longitudinal direction of the sanitary napkin 1. This contributes, during use of the sanitary napkin 1, to a secure and comfortable fit for the user, while maintaining adequate absorption properties.

Figure 3 shows an embodiment in which the outer edges 17, 18 of the wings 9, 10 are generally straight and parallel in relation to the longitudinal axis Y1. According to a further embodiment, however, the outer edges can extend a further distance over the main body 2, i.e. so that they extend over the longitudinal axis Y1. It should be noted that, irrespective of the actual positions of the outer edges 14, 15, it is advantageous if the adhesive areas 11, 12 are positioned - when the wings 9, 10 are folded - in a manner so that they overlap and cooperate with the adhesive sections 18 on the rear side of the main body 2 during use.

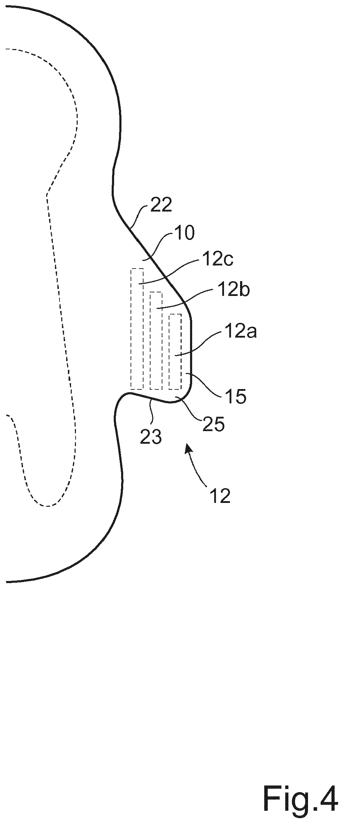

According to a further embodiment which is shown in Figure 4, the fastening means 12 can be formed by a number of relatively small areas 12a, 12b, 12c which together cover an area of the corresponding wing 10. These small areas 12a, 12b, 12c can be rectangular, as shown in Figure 4, or can be of any other suitable form in order to cover a suitable area of the corresponding wing 10. As an alternative to the embodiment in Figure 4, the small areas can be arranged so that the extend for example in a direction which is transverse to the longidudinal axis Y1. Also, in a manner which is similar to Figure 1, the small areas 12a, 12b, 12c shown in Figure 4 are placed so that a grip tab 25 is formed. This is due to the fact that the third angle α3 (see also Figure 1) is greater than 90°.

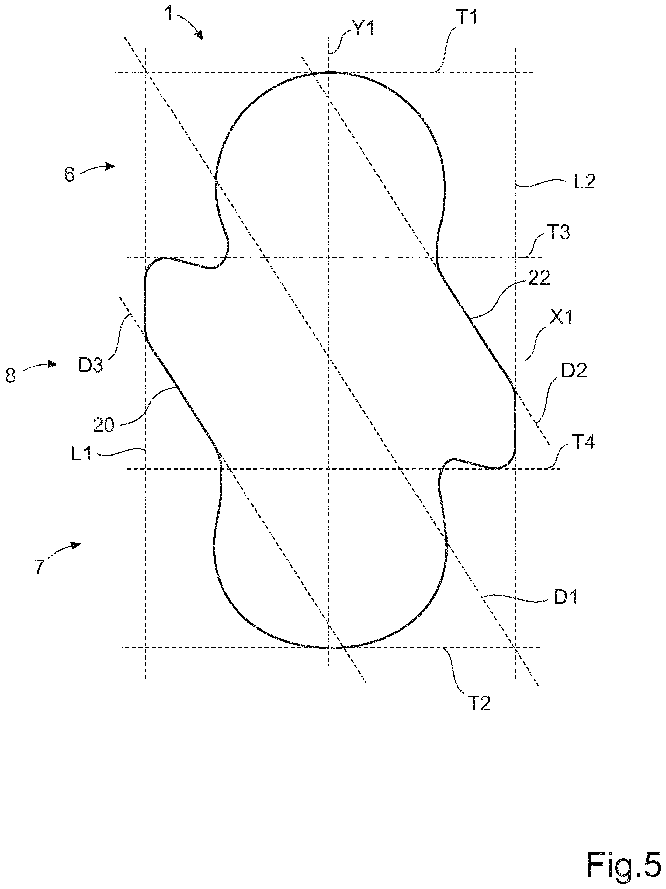

Figure 5 shows an embodiment which describes certain geometric properties of the invention, as will now be described. Initially, a first longitudinal line L1 is defined. With reference to Figures 1 and 5, the first longitudinal line L1 is parallel with the longitudinal axis Y1 and also extends through the outer edge 14 of the first wing 9. Furthermore, a second longitudinal line L2 is defined, which is also parallel with the longitudinal axis Y1 and extends through the outer edge 15 of the second wing 10.

Also, a front transversal line T1 is shown in Figure 5. This front transversal line T1 is parallel with the transversal line X1 and extends through a front edge of the front portion 6 of the sanitary napkin 1. Similarly, a back transversal line T2 is defined, which is also parallel with the transversal axis X1 and extends through a rear edge of the back portion 7 of the sanitary napkin 1.

Furthermore, a diagonal axis D1 is shown in Figure 5. The diagonal axis D1 extends through the point where the first longitudinal line L1 meets the front transversal line T1 and through the point where the second longitudinal line L2 meets the back transversal line T2. Also, a second diagonal line D2 extends along the fourth edge section 22 as mentioned above (and shown in Figure 1) and a third diagonal line D3 extends along the second edge section 20 as also mentioned above.

According to the embodiment, the second diagonal line D2, i.e. the fourth edge section 22 and the third diagonal line D3, i.e. the second edge section 20, are substantially parallel with the diagonal axis D. More precisely, the angle which can be defined between the second diagonal line D2 (or the third diagonal line D3) and the diagonal axis D is not more than within the interval ± 10°, preferably not more than ± 5°.

With regard to the geometry of the main body 2, it should be noted that the section of the napkin 1 referred to as the crotch portion 8 extends between a third transversal line T3 and a fourth transversal line T4, as shown in Figure 5. More precisely, the third transversal line T3 extends through a front edge 30 of the first wing 9, whereas the fourth transversal line T4 extends through a rear edge 31 of the secong wing 10.

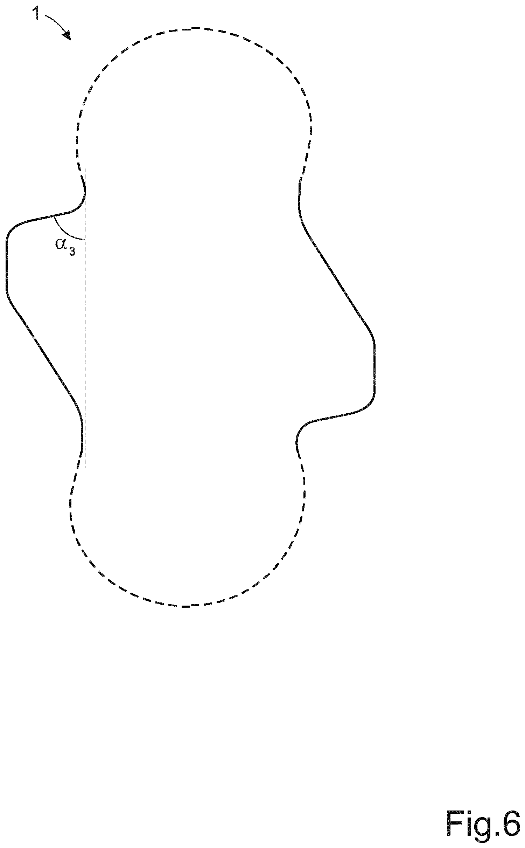

Figures 6-9 show further embodiments which merely represent possible exemplary executions and are not part of the present invention.

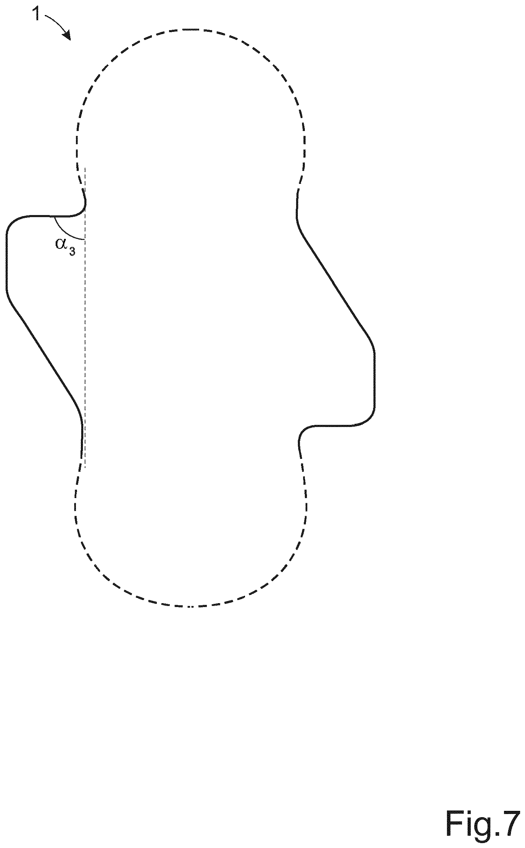

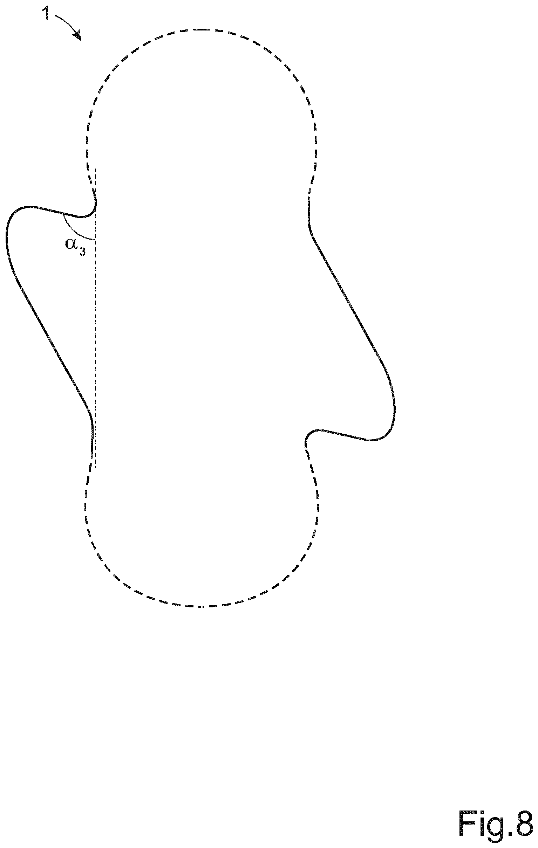

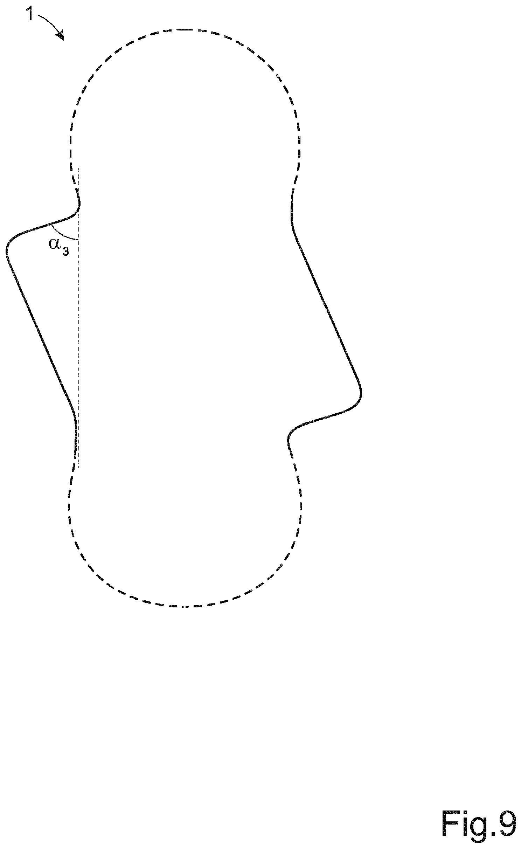

Firstly, it should be noted that Figure 6 shows a design of the napkin in which the angle corresponding to α3 (see Figure 1) is less than 90°, meaning that the grip tab (as formed in the embodiment of Figure 1) is not formed in the same manner in the embodiment of Figure 6. Furthermore, Figure 7 shows a design of the napkin in which the angle corresponding to α3 (see Figure 1) is 90°. Furthermore, Figure 8 and Figure 9 shows designs of the napkin having a slightly rounded outer edge of each wing.

A process for manufacturing the sanitary napkin 1 described above comprises a number of steps which will now be described. Initially, a material which constitutes the absorbent core 4 is sandwiched between the backsheet 2 and the topsheet 3 in a manner which is known as such. In this manner, the main body 2 is formed. Subsequently, the first wing 9 and the second wing 10 are formed in a manner so that they extend outwardly from the main body 2, in opposite directions and generally along said transversal axis X1, as described above. Furthermore, the wings 9, 10 are provided with fastening means 11, 12 for fastening the wings 9, 10 to a garment when they are folded over the main body 2. Furthermore, the manufacturing method comprises the step of arranging the wings 9, 10 so as to extend generally along a common straight line 13 which defines an angle α1 with respect to said transversal axis X1 and which exceeds 0 degrees but is less than 45 degrees, and also the step of forming each wing 9, 10 with an outer edge 14, 15 which is generally straight and parallel to the longitudinal axis Y1.

After manufacturing of the sanitary napkin 1, it is packaged by first folding the back portion 7 and the front portion 6 over the crotch portion 8, after which the folded napkin 1 is wrapped in an individual wrapping and stored in a box. Alternatively, the front portion 6 can be folded first over the crotch portion 8, after which the back portion 7 is folded over the crotch portion 8 and the folded napkin 1 is then wrapped.

As mentioned above with reference to Figure 3, the wings 9, 10 are folded along the first folding edge 26 during the manufacturing step. During manufacturing, however, the sanitary napkin 1 can alternatively be folded in a manner in which the wings 9, 10 and parts of the edge region 28 are folded along the core edge 27. This means that the width of the folded product can be made less than according to prior art, which is an advantage since the quantity of some of the material needed - in particular the release paper 19 and the wrapping material - can be reduced.

The invention is not limited to the embodiment but can be varied within the scope of the appended claims. For example, the particular shape of the wings 9, 10 may vary within the scope of the claims. Also, the materials and dimensions used for the different layers forming the absorbent article 1 can be varied, as indicated above. Also, all embodiments of wings and other geometries can be reversed as compared with the shown embodiments.

## Claims

1. An absorbent article (1) comprising a main body (2) having a liquid-permeable topsheet (3), a liquid-impermeable backsheet (4) and an absorbent core (5) sandwiched between said topsheet (3) and said backsheet (4), said main body (2) being arranged along a longitudinal axis (Y1) and a transversal axis (X1) extending in a perpendicular direction in relation to the longitudinal axis (Y1), said main body (2) defining a front portion (6), a back portion (7) and a crotch portion (8); wherein said article (1) comprises a first wing (9) and a second wing (10) extending outwardly from said crotch portion (8) of the main body (2) in opposite directions in an asymmetric manner with reference to the longitudinal axis (Y1), and generally along said transversal axis (X1), said wings (9, 10) being provided with fastening means (11, 12) configured for fastening said wings (9, 10) to a garment when being folded over said main body (2) and towards said longitudinal axis (Y1), **characterized in that** each wing has a first edge section (20) extending generally along a first straight line (20a) to an outer edge (14, 15) and a second edge section (21) extending generally along a second straight line (21a) to the outer edge (14, 15), wherein the first line (20a) defines a first angle (α2) with respect to the longitudinal axis (Y1) and wherein the second line (21a) defines a second angle (α3) with respect to the longitudinal axis (Y1), the magnitude of said angles (α2, α3) being chosen so that α2<α3, wherein said second angle (α3) is greater than 90° and wherein said wings (9, 10) are formed with an area (24, 25) between said fastening means (11, 12) and a further edge (21, 23) which constitutes a grip tab, and wherein said area (24, 25) is not covered with any adhesive.

2. An absorbent article (1) according to claim 1, wherein the magnitude of said first angle (α2) is chosen so that: 20° < α2 < 55°, and/or the magnitude of said second angle (α3) is chosen so that: 70° < α3 < 110°.

3. An absorbent article (1) according to any one of the preceding claims, wherein said wings (9, 10) extend generally along a further common straight line (13) which defines a third angle (α1) with respect to said transversal axis (X1) which exceeds 0° but which is less than 45°.

4. An absorbent article (1) according to any one of the preceding claims, wherein the outer edge (14, 15) is generally straight and parallel to the longitudinal axis (Y1).

5. An absorbent article (1) according to claim 3, wherein said straight line (13) extends between a first edge mid-point (16) on the outer edge (14) of the first wing (9) and a second edge mid-point (17) on the outer edge (15) on the second wing (10), said mid-points (16, 17) being positioned halfway along said outer edges (14, 15) and defining outermost points of said wings (9, 10).

6. An absorbent article (1) according to any one of the preceding claims, wherein a first distance (d1), as defined between the outer edge (15) of each wing (10) and the longitudinal axis (Y1), is in the range of 40-100 mm.

7. An absorbent article (1) according to any one of the preceding claims, wherein at least a part of each longitudinal side of the main body (2) defines a first wing folding edge (26a) where each wing (9, 10) meets the main body (2) and along which each wing (9, 10) is folded during use of the article (1).

8. An absorbent article (1) according to claim 7, wherein a second distance (d2), as defined between the first wing folding edge (26) and the corresponding outer edge (15) is in the range of 20-60 mm.

9. An absorbent article (1) according to any one of the preceding claims, wherein a second wing folding edge (26a) is defined where each wing (9, 10) can be folded during use of the article (1), said second wing folding edge (26a) having a length which is within the interval 10-80% of the full length of the main body, and preferably 25-65% of the full length of the main body (2).

10. An absorbent article (1) according to any one of the preceding claims, wherein each longitudinal side of the absorbent core (5) at least partly defines a core edge (27) which is generally parallel to the longitudinal axis (Y1).

11. An absorbent article (1) according to claim 10, wherein a third distance (d3), as defined between the core edge (27) and the corresponding outer edge (15) is in the range of 29-69 mm.

12. An absorbent article (1) according to claim 9 or 10, wherein a fourth distance (d4) as defined between the core edge (27) and the longitudinal axis (Y1) is in the range of 10-50 mm.

13. An absorbent article (1) according to any one of the preceding claims, wherein the absorbent core (5) generally follows the outer edge of the main body (2) and defines an edge region (28) of the main body which is generally equally wide along the entire periphery of the absorbent core (5), and/or said fastening means (11, 12) are in the form of one or more areas which support an adhesive material on each of said wing (9, 10), wherein the areas (11, 12) are preferably generally rectangular and positioned on each wing (9, 10) so that they are displaced in relation to each other in the longitudinal direction of the article, and/or the areas (11, 12) are arranged to generally extend along the entire transversal length of each corresponding wing (9, 10), and/or further comprising a second fastening means (18) positioned along the main body (2) on a garment-facing side of said backsheet (4).

14. A method for manufacturing an absorbent article (1) having a longitudinal extension along a longitudinal axis (Y1) and a transverse extension along a transverse axis (X1), said article (1) defining a front portion (6), a back portion (7) and a crotch region (8), said method comprising
- forming a main body (2) by providing a liquid-permeable topsheet (3), providing a liquid-impermeable backsheet (4) and sandwiching an absorbent body (5) between said topsheet (3) and said backsheet (4);
- forming a first wing (9) and a second wing (10) configured so as to extend outwardly from said main body (2) in opposite directions in an asymmetric manner with reference to the longitudinal axis (Y1) and generally along said transversal axis (X1); and
- providing said wings (9, 10) with fastening means (11, 12) for fastening said wings (9, 10) to a garment when being folded over said main body (2) and towards said longitudinal axis (Y1);
**characterized in that** the method comprises the following steps:
- forming each wing with a first edge section (20) extending generally along a first straight line (20a) to an outer edge (14, 15) and a second edge section (21) extending generally along a second straight line (21a) to the outer edge (14, 15), wherein the first line (20a) defines a first angle (α2) with respect to the longitudinal axis (Y1) and wherein the second line (21a) defines a second angle (α3) with respect to the longitudinal axis (Y1), and
- choosing the magnitude of said angles (α2, α3) so that α2<α3 and so that α3 > 90°; and
- forming said wings (9, 10) with an area (24, 25) between said fastening means (11, 12) and a further edge (21, 23) which constitutes a grip tab tab, wherein said area (24, 25) is not covered with any adhesive.

15. A method according to claim 14, wherein the magnitude of said first angle (α2) is chosen so that: 20° < α2 < 55°, and/or the magnitude of said second angle (α3) is chosen so that: 70° < α2 < 110°, and/or wherein the method further comprises the following steps:
- arranging said wings (9, 10) so as to extend generally along a common straight line (13) which defines an angle (α1) with respect to said transversal axis (X1) which exceeds 0 degrees but is less than 45 degrees, and
- forming the outer edge (14, 15) generally straight and parallel to the longitudinal axis (Y1).

## Patentansprüche

1. Ein absorbierender Artikel (1), der einen Hauptkörper (2) mit einer flüssigkeitsdurchlässigen Oberschicht (3), einer flüssigkeitsundurchlässigen Unterschicht (4) und einem absorbierenden Kern (5) umfasst, der zwischen der besagten Oberschicht (3) und der besagten Unterschicht (4) angeordnet ist, wobei der besagte Hauptkörper (2) entlang einer Längsachse (Y1) und einer Querachse (X1) angeordnet ist, die sich in eine senkrechte Richtung in Bezug auf die Längsachse (Y1) erstreckt, wobei der besagte Hauptkörper (2) einen Vorderteil (6), einen Rückenteil (7) und einen Schrittteil (8) definiert; wobei der besagte Artikel (1) einen ersten Flügel (9) und einen zweiten Flügel (10) umfasst, die sich von dem besagten Schrittteil (8) des Hauptkörpers (2) in entgegengesetzte Richtungen asymmetrisch in Bezug auf die Längsachse (Y1) und im Allgemeinen entlang der besagten Querachse (X1) nach außen erstrecken, wobei die besagten Flügel (9, 10) mit Befestigungsmitteln (11, 12) versehen sind, die konfiguriert sind, um die besagten Flügel (9, 10) an einem Kleidungsstück zu befestigen, wenn sie über den besagten Hauptkörper (2) und in Richtung der besagten Längsachse (Y1) gefaltet werden, **gekennzeichnet dadurch, dass** jeder Flügel einen ersten Kantenabschnitt (20) hat, der sich im Allgemeinen entlang einer ersten geraden Linie (20a) zu einer Außenkante (14, 15) erstreckt, und einen zweiten Kantenabschnitt (21), der sich im Allgemeinen entlang einer zweiten geraden Linie (21a) zu der Außenkante (14, 15) erstreckt, wobei die erste Linie (20a) einen ersten Winkel (α2) in Bezug auf die Längsachse (Y1) definiert und wobei die zweite Linie (21a) einen zweiten Winkel (α3) in Bezug auf die Längsachse (Y1) definiert, wobei die Größe der besagten Winkel (α2, α3) so gewählt wird, dass α2<α3, wobei der besagte zweite Winkel (α3) größer als 90° ist und wobei die besagten Flügel (9, 10) mit einem Bereich (24, 25) zwischen den besagten Befestigungsmitteln (11, 12) und einer weiteren Kante (21, 23) gebildet sind, die eine Grifflasche bildet, und wobei der besagte Bereich (24, 25) nicht mit Klebstoff bedeckt ist.

2. Absorbierender Artikel (1) nach Anspruch 1, wobei die Größe des besagten ersten Winkels (α2) so gewählt ist, dass: 20° < α2 < 55° ist, und/oder die Größe des besagten zweiten Winkels (α3) so gewählt ist, dass: 70° < α3 < 110°.

3. Absorbierender Artikel (1) nach einem der vorhergehenden Ansprüche, wobei sich die besagten Flügel (9, 10) im Allgemeinen entlang einer weiteren gemeinsamen geraden Linie (13) erstrecken, die einen dritten Winkel (α1) in Bezug auf die besagte Querachse (X1) definiert, der 0° überschreitet, aber kleiner als 45° ist.

4. Absorbierender Artikel (1) nach einem der vorhergehenden Ansprüche, wobei die Außenkante (14, 15) im Allgemeinen gerade und parallel zu der Längsachse (Y1) ist.

5. Absorbierender Artikel (1) nach Anspruch 3, wobei sich die besagte gerade Linie (13) zwischen einem ersten Kantenmittelpunkt (16) an der Außenkante (14) des ersten Flügels (9) und einem zweiten Kantenmittelpunkt (17) an der Außenkante (15) des zweiten Flügels (10) erstreckt, wobei die besagten Mittelpunkte (16, 17) auf halbem Weg entlang der besagten Außenkanten (14, 15) angeordnet sind und die äußersten Punkte der besagten Flügel (9, 10) definieren.

6. Absorbierender Artikel (1) nach einem der vorhergehenden Ansprüche, wobei eine erste Distanz (d1), wie zwischen der Außenkante (15) jedes Flügels (10) und der Längsachse (Y1) definiert, im Bereich von 40-100 mm liegt.

7. Absorbierender Artikel (1) nach einem der vorhergehenden Ansprüche, wobei mindestens ein Teil jeder Längsseite des Hauptkörpers (2) eine erste Flügelfaltkante (26a) definiert, an der jeder Flügel (9, 10) auf den Hauptkörper (2) trifft und entlang der jeder Flügel (9, 10) während des Gebrauchs des Artikels (1) gefaltet wird.

8. Absorbierender Artikel (1) nach Anspruch 7, wobei eine zweite Distanz (d2), wie zwischen der ersten Flügelfaltkante (26) und der entsprechenden Außenkante (15) definiert, im Bereich von 20-60 mm liegt.

9. Absorbierender Artikel (1) nach einem der vorhergehenden Ansprüche, wobei eine zweite Flügelfaltkante (26a) definiert ist, an der jeder Flügel (9, 10) während des Gebrauchs des Artikels (1) gefaltet werden kann, wobei die besagte zweite Flügelfaltkante (26a) eine Länge aufweist, die innerhalb des Intervalls von 10-80 % der vollen Länge des Hauptkörpers und vorzugsweise 25-65 % der vollen Länge des Hauptkörpers (2) liegt.

10. Absorbierender Artikel (1) nach einem der vorhergehenden Ansprüche, wobei jede Längsseite des absorbierenden Kerns (5) zumindest teilweise eine Kernkante (27) definiert, die im Allgemeinen parallel zu der Längsachse (Y1) ist.

11. Absorbierender Artikel (1) nach Anspruch 10, wobei eine dritte Distanz (d3), wie zwischen der Kernkante (27) und der entsprechende Außenkante (15) definiert, im Bereich von 29-69 mm liegt.

12. Absorbierender Artikel (1) nach Anspruch 9 oder 10, wobei eine vierte Distanz (d4), wie zwischen der Kernkante (27) und der Längsachse (Y1) definiert, im Bereich von 10-50 mm liegt.

13. Absorbierender Artikel (1) nach einem der vorhergehenden Ansprüche, wobei der absorbierende Kern (5) im Allgemeinen der Außenkante des Hauptkörpers (2) folgt und einen Kantenbereich (28) des Hauptkörpers definiert, der im Allgemeinen entlang des gesamten Umfangs des absorbierenden Kerns (5) gleich breit ist, und/oder die besagten Befestigungsmittel (11, 12) die Form eines oder mehrerer Bereiche haben, die ein Klebematerial auf jedem der besagten Flügel (9, 10) tragen, wobei die Bereiche (11, 12) vorzugsweise im Allgemeinen rechteckig sind und auf jedem Flügel (9, 10) so angeordnet sind, dass sie in Bezug zueinander in der Längsrichtung des Artikels versetzt sind, und/oder die Bereiche (11, 12) angeordnet sind, um sich im Allgemeinen entlang der gesamten Querlänge jedes entsprechenden Flügels (9, 10) zu erstrecken, und/oder ferner ein zweites Befestigungsmittel (18) umfassen, das entlang des Hauptkörpers (2) auf einer der Kleidung zugewandten Seite der besagten Unterschicht (4) angeordnet ist.

14. Verfahren zum Herstellen eines absorbierenden Artikels (1) mit einer Längserstreckung entlang einer Längsachse (Y1) und einer Quererstreckung entlang einer Querachse (X1), wobei der besagte Artikel (1) einen Vorderteil (6), einen Rückenteil (7) und einen Schrittteil (8) definiert, wobei das Verfahren Folgendes umfasst
- Bilden eines Hauptkörpers (2) durch Bereitstellen einer flüssigkeitsdurchlässigen Oberschicht (3), Bereitstellen einer flüssigkeitsundurchlässigen Unterschicht (4) und Anordnen eines absorbierenden Körpers (5) zwischen der besagten Oberschicht (3) und der besagten Unterschicht (4);
- Bilden eines ersten Flügels (9) und eines zweiten Flügels (10), die so konfiguriert sind, dass sie sich von dem besagten Hauptkörper (2) in entgegengesetzte Richtungen asymmetrisch in Bezug auf die Längsachse (Y1) und im Allgemeinen entlang der besagten Querachse (X1) nach außen erstrecken; und
- Versehen der besagten Flügel (9, 10) mit Befestigungsmitteln (11, 12), um die besagten Flügel (9, 10) an einem Kleidungsstück zu befestigen, wenn sie über den besagten Hauptkörper (2) und in Richtung der besagten Längsachse (Y1) gefaltet werden;
**gekennzeichnet dadurch, dass** das Verfahren die folgenden Schritte umfasst:
- Bilden jedes Flügels mit einem ersten Kantenabschnitt (20), der sich im Allgemeinen entlang einer ersten geraden Linie (20a) zu einer Außenkante (14, 15) erstreckt, und einem zweiten Kantenabschnitt (21), der sich im Allgemeinen entlang einer zweiten geraden Linie (21a) zu der Außenkante (14, 15) erstreckt, wobei die erste Linie (20a) einen ersten Winkel (α2) in Bezug auf die Längsachse (Y1) definiert und wobei die zweite Linie (21a) einen zweiten Winkel (α3) in Bezug auf die Längsachse (Y1) definiert, und
- Wählen der Größe der besagten Winkel (α2, α3), so dass α2<α3 und so dass α3 > 90°; und
- Bilden der besagten Flügel (9, 10) mit einem Bereich (24, 25) zwischen den besagten Befestigungsmitteln (11, 12) und einer weiteren Kante (21, 23), die eine Grifflasche bildet, wobei der besagte Bereich (24, 25) nicht mit Klebstoff bedeckt ist.

15. Verfahren nach Anspruch 14, wobei die Größe des besagten ersten Winkels (α2) so gewählt wird, dass: 20° < α2 < 55°, und/oder die Größe des besagten zweiten Winkels (α3) so gewählt wird, dass: 70° < α2 < 110°, und/oder wobei das Verfahren ferner die folgenden Schritte umfasst:
- Anordnen der besagten Flügel (9, 10), so dass sie sich im Allgemeinen entlang einer gemeinsamen geraden Linie (13) erstrecken, die einen Winkel (α1) in Bezug auf die besagte Querachse (X1) definiert, der 0 Grad überschreitet, aber kleiner als 45 Grad ist, und
- Bilden der Außenkante (14, 15) im Allgemeinen gerade und parallel zu der Längsachse (Y1).

## Revendications

1. Article absorbant (1) comprenant un corps principal (2) ayant une feuille supérieure perméable aux liquides (3), une feuille inférieure imperméable aux liquides (4) et un coeur absorbant (5) intercalé entre ladite feuille supérieure (3) et ladite feuille inférieure (4), ledit corps principal (2) étant disposé le long d'un axe longitudinal (Y1) et d'un axe transversal (X1) s'étendant dans une direction perpendiculaire par rapport à l'axe longitudinal (Y1), ledit corps principal (2) définissant une partie avant (6), une partie arrière (7) et une partie d'entrejambe (8) ; où ledit article (1) comprend une première aile (9) et un deuxième aile (10) s'étendant vers l'extérieur de ladite partie d'entrejambe (8) du corps principal (2) dans des directions opposées de manière asymétrique par rapport à l'axe longitudinal (Y1), et généralement le long dudit axe transversal (X1), lesdites ailes (9, 10) étant pourvues de moyens de fixation (11, 12) configurés pour fixer lesdites ailes (9, 10) à un vêtement lorsqu'elles sont repliées sur ledit corps principal (2) et vers ledit axe longitudinal (Y1), **caractérisé en ce que** chaque aile a une première section de bord (20) s'étendant généralement le long d'une première ligne droite (20a) à un bord extérieur (14, 15) et une deuxième section de bord (21) s'étendant généralement le long d'une deuxième ligne droite (21a) au bord extérieur (14, 15), où la première ligne (20a) définit un premier angle (α2) par rapport à l'axe longitudinal (Y1) et où la deuxième ligne (21a) définit un deuxième angle (α3) par rapport à l'axe longitudinal (Y1), l'amplitude desdits angles (α2 ; α3) étant choisie de sorte que α2 < α3, où ledit deuxième angle (α3) est supérieur à 90° et où lesdites ailes (9, 10) sont formées avec un zone (24, 25) entre lesdits moyens de fixation (11, 12) et un autre bord (21, 23) qui constitue une languette de préhension, et où ladite zone (24, 25) n'est recouverte d'aucun adhésif.

2. Article absorbant (1) selon la revendication 1, où l'amplitude dudit premier angle (α2) est choisie de sorte que : 20° < α2 < 55°, et/ou l'amplitude dudit deuxième angle (α3) est choisie de sorte que : 70° < α3 < 110°.

3. Article absorbant (1) selon l'une quelconque des revendications précédentes, où lesdites ailes (9, 10) s'étendent généralement le long d'une ligne supplémentaire droite commune (13) qui définit un troisième angle (α1) par rapport audit axe transversal (X1) qui excède 0 degré mais qui est inférieur à 45 degrés.

4. Article absorbant (1) selon l'une quelconque des revendications précédentes, où le bord extérieur (14, 15) est généralement droit et parallèle à l'axe longitudinal (Y1).

5. Article absorbant (1) selon la revendication 3, où ladite ligne droite (13) s'étend entre un premier point médian de bord (16) sur le bord extérieur (14) de la première aile (9) et un deuxième point médian de bord (17) sur le bord extérieur (15) de la deuxième aile (10), lesdits points médians (16, 17) étant positionnés à mi-chemin le long desdits bords extérieurs (14, 15) et définissant les points les plus extérieurs desdites ailes (9, 10).

6. Article absorbant (1) selon l'une quelconque des revendications précédentes, où une première distance (d1), comme définie entre le bord extérieur (15) de chaque aile (10) et l'axe longitudinal (Y1), est de l'ordre de 40-100 mm.

7. Article absorbant (1) selon l'une quelconque des revendications précédentes, où au moins une partie de chaque côté longitudinal du corps principal (2) définit un premier bord de pliage d'aile (26a) où chaque aile (9, 10) rencontre le corps principal (2) et le long duquel chaque aile (9, 10) est pliée lors de l'utilisation de l'article (1).

8. Article absorbant (1) selon la revendication 7, où une deuxième distance (d2), comme définie entre le premier bord de pliage d'aile (26) et le bord extérieur correspondant (15) est de l'ordre de 20-60 mm.

9. Article absorbant (1) selon l'une quelconque des revendications précédentes, où un deuxième bord de pliage d'aile (26a) est défini où chaque aile (9, 10) peut être pliée lors de l'utilisation de l'article (1), ledit deuxième bord de pliage d'aile (26a) ayant une longueur qui est comprise dans l'intervalle de 10-80% de la longueur totale du corps principal, et de préférence de 25-65% de la longueur totale du corps principal (2).

10. Article absorbant (1) selon l'une quelconque des revendications précédentes, où chaque côté longitudinal du coeur absorbant (5) définit au moins partiellement un bord du coeur (27) qui est généralement parallèle à l'axe longitudinal (Y1).

11. Article absorbant (1) selon la revendication 10, où une troisième distance (d3), comme définie entre le bord du coeur (27) et le bord extérieur correspondant (15) est de l'ordre de 29-69 mm.

12. Article absorbant (1) selon la revendication 9 ou 10, où une quatrième distance (d4) comme définie entre le bord du coeur (27) et l'axe longitudinal (Y1) est de l'ordre de 10-50 mm.

13. Article absorbant (1) selon l'une quelconque des revendications précédentes, où le coeur absorbant (5) suit généralement le bord extérieur du corps principal (2) et définit une région de bord (28) du corps principal qui est généralement de largeur égale sur toute la périphérie du coeur absorbant (5), et/ou lesdits moyens de fixation (11, 12) se présentent sous la forme d'une ou plusieurs zones qui supportent un matériau adhésif sur chacune de ladite aile (9, 10), où les zones (11, 12) sont de préférence généralement rectangulaires et positionnées sur chaque aile (9, 10) de sorte qu'elles sont décalées l'une par rapport à l'autre dans la direction longitudinale de l'article, et/ou les zones (11, 12) sont disposées pour s'étendre généralement sur toute la longueur transversale de chaque aile correspondante (9, 10), et/ou comprenant en outre un deuxième moyens de fixation (18) positionné le long du corps principal (2) sur un côté faisant face au vêtement de ladite feuille inférieure (4).

14. Procédé de fabrication d'un article absorbant (1) ayant une extension longitudinale le long d'un axe longitudinal (Y1) et une extension transversale le long d'un axe transversal (X1), ledit article (1) définissant une partie avant (6), une partie arrière (7) et une région d'entrejambe (8), ledit procédé comprenant
- former un corps principal (2) en fournissant une feuille supérieure perméable aux liquides (3), fournir une feuille inférieure imperméable aux liquides (4) et en prenant en sandwich un corps absorbant (5) entre ladite feuille supérieure (3) et ladite feuille inférieure(4) ;
- former une première aile (9) et une deuxième aile (10) configurées afin de s'étendre vers l'extérieur dudit corps principal (2) dans des directions opposées de manière asymétrique par rapport à l'axe longitudinal (Y1) et généralement le long dudit axe transversal (X1) ; et
- fournir lesdites ailes (9, 10) avec des moyens de fixation (11, 12) pour fixer lesdites ailes (9, 10) à un vêtement lorsqu'elles sont repliées sur ledit corps principal (2) et vers ledit axe longitudinal (Y1) ;
**caractérisé en ce que** le procédé comprend les étapes suivantes :
- former chaque aile avec une première section de bord (20) s'étendant généralement le long d'une première ligne droite (20a) jusqu'à un bord extérieur (14, 15) et une deuxième section de bord (21) s'étendant généralement le long d'une deuxième ligne droite (21a) jusqu'au bord extérieur (14, 15), où la première ligne (20a) définit un premier angle (α2) par rapport à l'axe longitudinal (Y1) et où la deuxième ligne (21a) définit un deuxième angle (α3) par rapport à l'axe longitudinal (Y1), et
- choisir l'amplitude desdits angles (α2 ; α3) de sorte que α2 < α3 et de sorte que α3 > 90° ; et
- former lesdites ailes (9, 10) avec une zone (24, 25) entre lesdits moyens de fixation (11, 12) et un autre bord (21, 23) qui constitue une languette de préhension, où ladite zone (24, 25) n'est recouverte d'aucun adhésif.

15. Procédé selon la revendication 14, où l'amplitude dudit premier angle (α2) est choisie de sorte que : 20° < α2 < 55°, et/ou l'amplitude dudit deuxième angle (α3) est choisie de sorte que : 70° < α2 < 110°, et/ou où le procédé comprend en outre les étapes suivantes :
- disposer lesdites ailes (9, 10) afin de s'étendre généralement le long d'une ligne droite commune (13) qui définit un angle (α1) par rapport audit axe transversal (X1) qui excède 0 degré mais est inférieur à 45 degrés, et
- former le bord extérieur (14, 15) généralement droit et parallèle à l'axe longitudinal (Y1).
